# NEUE EUROPÄISCHE PATENTSCHRIFT

(11) **EP 0 270 483 B2**
(45) Veröffentlichungstag und Bekanntmachung des Hinweises auf die Entscheidung über den Einspruch: **14.02.1996**
(45) Hinweis auf die Patenterteilung: 29.07.1992
(21) Anmeldenummer: 87730155.6
(22) Anmeldetag: 23.11.1987
(51) Int. Cl.: A61K 31/28

(54) **Verbesserte metallhaltige Pharmazeutika**
Pharmaceutical compounds containing metals
Préparations pharmaceutiques contenant des métaux

(30) Priorität: 28.11.1986 DE 3640708
(43) Veröffentlichungstag der Anmeldung: 08.06.1988
(73) Patentinhaber: SCHERING AKTIENGESELLSCHAFT, D-13342 Berlin (DE)
(72) Erfinder: Gries, Heinz, Dr., D-1000 Berlin 31 (DE); Speck, Ulrich, Prof. Dr., D-1000 Berlin 28 (DE); Weinmann, Hanns-Joachim, Dr., D-1000 Berlin 41 (DE); Niendorf, Hans Peter, Dr., D-1000 Berlin 33 (DE); Seifert, Wolfgang, Dr., D-1000 Berlin 19 (DE)

(56) Entgegenhaltungen:
- DE-A-33 024 103
- FR-A- 2 539 996
- GB-A- 736 432
- US-A- 3 663 688
- US-A- 4 478 816
- U. Speck, R. Felix, Radiology To-day (1985), 180-183.
- Runge et al.: Investigative Radiology (1984), 408-415.
- Nalbadian et al.: Annals New-York Academy of Sciences, (1959), 779-792.
- N. Sapeika, British Medical Journal,(1955), 167-169.
- Shapiro et al., Annals of New York Academy of Sciences (1959), 759-763
- H. Hart,USAEC, ORINS-12, (1956), 118-135
- James et al.: , J. de l'Association Canadienne des Radiologistes (1971), 136-143

## Beschreibung

Die Erfindung betrifft den in den Patentansprüchen gekennzeichneten Gegenstand.

Bald nach Entdeckung der Röntgenstrahlen wurden die unterschiedlichsten Substanzen als "Kontrastmittel" experimentell erprobt, um den nicht ausreichenden Kontrast von Körperflüssigkeiten und Weichteilgeweben zu verstärken (Barke, R., Röntgenkontrastmittel; Chemie, Physiologie, Klinik VEB Georg Thieme Leipzig, 1970). Als Röntgenstrahlen-absorbierende Bestandteile derartiger Kontrastmittel kamen schwere Elemente in Frage. Im Laufe eines langen Selektions- und Optimierungsprozesses waren schließlich nur Kontrastmittel auf der Basis von Jod (in fester organischer Bindung) oder Barium (als nahezu unlösliches Sulfat) übriggeblieben. Bariumsulfat wird ausschließlich zur Darstellung des Magen-Darm-Traktes angewandt, wobei es nicht in den Körper gelangt.

Mit der Entwicklung der Nuklearmedizin, d.h. der Verwendung radioaktiver Elemente zur Darstellung bestimmter Strukturen des Organismus, pathologischer Bezirke und besonders der Funktionsdiagnostik sowie zur Radiotherapie fanden eine Reihe von weiteren Metallen Eingang in die in vivo Diagnostik. Die in der Nuklearmedizin eingesetzten sogenannten Radiopharmaka enthalten entweder ein radioaktives Isotop des Jods (¹³¹J oder ¹²³J) oder bevorzugt ein Metall wie ^{99m} Technetium. Diese Elemente werden in vielen Fällen an eine organische Substanz gebunden oder, im Falle der radioaktiven Metallisotope, komplexiert verabreicht. Meist ist die Komplexierung der Metalle nicht so fest, daß nicht während des Aufenthaltes im Körper ein mehr oder minder großer Teil des Metalls aus der Bindung an das organische Molekül freigesetzt wird. Damit verliert das Metallion im allgemeinen seine erwünschten und durch die Komplexierung bedingten pharmakokinetischen und auch diagnostischen Eigenschaften, wird nur noch langsam ausgeschieden stört das an sich spezifische Verteilungsbild des noch gebundenen Isotops und kann seine inherent toxischen Eigenschaften entfalten.

Anfang der 80iger Jahre hat das Interesse an Metallkomplexen in der Diagnostik und Therapie weiter zugenommen. Mit der Entwicklung der Kernspintomographie stellte sich auch die Frage nach kontrastgebenden, d.h. signalbeenflussenden Substanzen, die dem Körper von außen zugeführt werden können. Solche Substanzen helfen, Erkrankungen früher und genauer zu erkennen. Als wirksames Prinzip wurden Komplexe paramagnetischer Metallionen eingeführt, die sich trotz relativ hoher Dosierung (d.h. mehrere Gramm Komplex die ca. 1 - 2 g Schwermetall enthalten) und rascher intravenöser Injektion als erstaunlich gut verträglich erwiesen haben (R. Felix, W. Schörner, M. Laniado, H.P Niendorf, C. Claussen, W. Fiegler, U. Speck; Radiology 156, 3: 681 - 688 (1985)). Besonders hervorzuheben ist die offensichtlich ausgezeichnete akute Verträglichkeit des Gadolinium-DTPA (Europäische Patentanmeldung 71564), des bisher in der klinischen Anwendung am weitesten fortgeschrittenen Präparates. Die äußerst geringe Zahl und die leichte Natur der durch Gadolinium-DTPA ausgelösten akuten Nebenwirkungen lassen es auch für eine Anwendung im Zusammenhang mit bestimmten Röntgentechniken als geeignet erscheinen. Für eine Reihe diagnostischer Probleme in der Kernspintomographie und ganz allgemein in der Röntgendiagnostik ergibt sich die Notwendigkeit höherer Dosierung und wiederholter Verabreichung. In diesem Zusammenhang ist der Frage der Langzeit-Verträglichkeit schwermetallhaltiger Substanzen große Aufmerksamkeit zu widmen.

Anders als bei Jod bei den jodhaltigen Röntgenkontrastmitteln sind die Zentralatome in den metallhaltigen Komplexverbindungen, die für die NMR-, Röntgen, Ultraschall- und Radiodiagnostik sowie für die Therapie geeignet sind, nicht kovalent gebunden. Die Bindung des Metallions unterliegt einem Gleichgewicht mit der Umgebung, das naturgemäß so weit wie möglich auf Seiten des Komplexes liegen sollte. Es kann aber nie eine vollständige Bindung erreicht werden. Zusätzlich ist zu beachten, daß die für die Komplexe angegebenen, z.T. sehr hohen Stabilitätskonstanten sich auf unphysiologisch hohe pH-Werte beziehen und für die Situation in vivo nicht zutreffen. Weiterhin kommt es in vivo zu einer Konkurrenz unterschiedlicher Ionen um die Bindung an den Komplexbildner, so daß die Wahrscheinlichkeit für die unerwünschte und unter Umständen gefährliche Freisetzung von Schwermetallionen im Organismus steigt.

Die Gefahr wird noch umso größer
- je höher die Dosierung des Schwermetallkomplexes ist
- je häufiger der Komplex angewandt wird
- je länger er im Körper verweilt
- je chemisch oder metabolisch instabiler der Komplexbildner ist und
- je stärker er in die Zellen des Körpers gelangt.

Andererseits sind gerade die für die Diagnostik sowie Radiotherapie bestimmter Arten pathologischer Veränderungen erwünschten gewebsspezifischen Komplexe, z.B. auch die an Bio- oder Makromoleküle gebundenen Komplexe, durch eine im Vergleich zu dem Gadoliniun-DTPA längere und mehr intrazelluläre Aufenthaltsdauer im Körper gekennzeichnet.

Es besteht daher für vielfältige Zwecke ein Bedarf an besser verträglichen pharmazeutischen Mitteln, bei denen eine Freisetzung des betreffenden Schwermetallions aus der Komplexbindung möglichst weitgehend verhindert ist.

Der Erfindung liegt somit die Aufgabe zugrunde, die in-vivo-Verträglichkeit von pharmazeutischen Mittel für die NMR-Diagnostik zu verbessern. Diese Aufgabe wird erfindungsgemäß gelöst durch Zusatz eines oder mehrerer Komplexbildner(s) oder/und eines oder mehrerer schwächeren/schwächerer Metallkomplexe(s) oder ihrer Gemische in einer Menge von 0,1 - 10 Mol% oder max. 50 mMol/l zu pharmazeutischen Mitteln für die NMR-Diagnostik enthaltend mindestens eine für die NMR-Diagnostik geeignete, ein paramagnetisches Metall, ausgenommen Chrom, enthaltende Komplexverbindung.

Der Komplexbildner kann dabei in allen drei Fällen (Diagnostikum bzw. Therapeutikum, Zusatz Komplexbildner, Zusatz schwächerer Metallkomplex) identisch oder auch verschieden sein. In Frage kommen z.B. die in den Patentanmeldungen EP 71.564 (z.B. Ethylen diamintetraessigsäure EDTA, Diethylentriaminpentaessigsäure DTPA), DE-OS 3401052 (z.B. 1,4, 7,10-Tetraazacyclododecan-N,N',N'',N'''-tetraessigäsure DOTA, trans-1,2-Cyclohexylendiamin-N,N,N',N'-tetraessigsäure),EP 130934 (z.B. N⁶-Carboxymethyl-N³,N⁹-[2,3-dihydroxy-N-methylpropylcarbamoylmethyl] -3,6,9-triazaundecandisäure) aufgeführten Komplexbildner sowie z.B. N⁶-Carboxymethyl-N³,N⁹-bis (methylcarbamoylmethyl)-3,6,9-triazaundecandisäure, N³,N⁶-Bis-(carboxymethyl)-N⁹-3-oxapentamethylen-carbamoylmethyl-3,6,9-triazaundecandisäure, N³,N⁶-Bis (carboxymethyl)-N⁹ [3,3-bis (dihydroxyphosphoryl)-3-hydroxypropyl-carbamoylmethyl]-3,6,9-triazaundecandisäure.

Unter schwächeren Metallkomplexen sind solche zu verstehen, die eine relativ geringere Stabilitätskonstante besitzen; bevorzugt sind solche, die als Zentralatom ein Metallion von natürlicherweise im Organismus vorhandenen Elemente wie Calcium, Magnesium, Zink und Eisen besitzen.

Die Komplexbildner und Metallkomplexe können als physiologisch unbedenkliche Salze anorganischer (z.B. Kalium-, Natrium-,Lithium-hydroxid), organischer (z.B. primäre, sekundäre, tertiäre Amine wie z.B. Ethanolamin, Morpholin, Glucamin, N-Methyl-, N,N-Dimethylglucamin) Basen, basischer Aminosäuren und Aminosäureamiden (z.B. Lysin, Arginin, Ornithin bzw. Säuren (z.B. Glucuronsäure, Essigsäure) eingesetzt werden.

Die Herstellung von Additiven soll im folgenden beispielhaft beschrieben werden:

### 1) Calcium-Dinatriumsalz der 1,4,7,10 - Tetraazacycloclodecan-N,N',N'',N'''-tetraessigsäure (DOTA)

40,40 g (0,1 Mol) DOTA (Parish Chemical Comp.) werden mit 10,0 g (0,1 Mol) Calciumcarbonat in 100 ml Wasser bis zur Beendigung der Gasentwicklung am Rückfluß erhitzt. Dann stellt man durch Zugabe von 200 ml einer 1 n - Natronlauge eine neutrale Salzlösung her, die im Vakuum zur Trockne eingeengt wird. Man erhält 50,5 g eines Monohydrats als weißes Pulver mit einem oberhalb 250° C liegendem Zersetzungspunkt.

| Analyse (bezogen auf wasserfreie Substanz): | | | | |
|---|---|---|---|---|
| C 39,50 | H 4,97 | N 11,52 | Ca 8,24 | (berechnet) |
| C 39,65 | H 5,05 | N 11,30 | Ca 8,18 | (gefunden) |

In analoger Weise erhält man aus DOTA, Zinkcarbonat und Natronlauge das Zink-Dinatriumsalz der DOTA.

### 2) N⁶-Carboxymethyl-N³,N⁹-bis-(methylcarbamoylmethyl)-3,6,9-triazaundecandisäure

17,9 g (50 mMol) 1,5-Bis(2,6-dioxomorpholino)-3-azapentan-3-essigsäure werden in 50 ml Wasser mit 100 ml einer wässrigen molaren Lösung von Methylamin versetzt. Es wird 12 Stunden bei Raumtemperatur gerührt und die schwach gelbe Lösung durch Filtration über Aktivkohle entfärbt. Nach Einengen im Vakuum erhält man 20,5 g (= 98 % der Theorie) eines weißen hygroskopischen Pulvers vom Schmelzpunkt 78 - 82° C.

| Analyse (bezogen auf wasserfreie Substanz): | | | |
|---|---|---|---|
| C 45,81 | H 6,97 | N 16,70 | (berechnet) |
| C 45,62 | H 7,03 | N 16,52 | (gefunden) |

### 3 a) N³-(2,6-Dioxomorpholinoethyl)-N⁶-(ethoxycarbonylmethyl)-3,6-diazaoctandisäure.

Eine Suspension von 21,1 g (50 mMol) N³,N⁶-Bis-(carboxymethyl)-N⁹-(ethoxycarbonylmethyl)-3,6,9-triazaundecandisäure (J. Pharm.Sci. 68, 1979, 194) in 250 ml Essigsäureanhydrid läßt man nach Zugabe von 50 ml Pyridin drei Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40° C -erhält man 18,0 g (= 89 % der Theorie) eines weißen Pulvers vom Schmelzpunkt 195° - 196°C.

| Analyse: | | | |
|---|---|---|---|
| C 47,64 | H 6,25 | N 10,42 | (berechnet) |
| C 47,54 | H 6,30 | N 10,22 | (gefunden) |

### 3 b) Tetranatriumsalz der N³,N⁶-Bis-(carboxymethyl)-N⁹-3-oxapentamethylencarbamoylmethyl-3,6,9-triazaundecaddisäure.

2,42 g (6 mMol) der unter a) enthaltenen Verbindung werden in 30 ml Dimethylformamid suspendiert. Dann gibt man bei -5°C 3,04 g (30 mMol) Triethylamin und 0,52 ml (6 mMol) Morpholin zu, läßt bei dieser Temperatur 2 Stunden, dann noch über Nachtnachrühren, engt die Lösung im Vakuum zur Trockne ein und verrührt den Rückstand mit 100 ml Diisopropylether. Nach dem Absaugen und Trocknen wird die Substanz in 40 ml Wasser und 24 ml 0,1 n - Natronlauge gelöst. Man läßt 2 Stunden bei Raumtemperatur rühren und engt die Lösung im Vakuum zur Trockne ein. Der Rückstand wird mit 10 ml Isopropanol verrührt, abgesaugt, mit Isopropanol gewaschen und bei 60° C im Vakuum getrocknet. Man erhält 2,85 g (= 86 % der Theorie) als weißes Pulver mit einem oberhalb 250° C liegendem Zersetzungspunkt.

| Analyse (bezogen auf wasserfreie Substanz): | | | |
|---|---|---|---|
| C 39,28 | H 4,76 | N 10,18 | (berechnet) |
| C 39,11 | H 5,01 | N 10,23 | (gefunden) |

### 4) N³,N⁶-Bis (carboxymethyl)-N⁹ [3,3-bis(dihydroxyphosphoryl) -3-hydroxypropylcarbamoylmethyl] - 3,6,9-triazaundecandisäure.

2,35 g (10 mMol) 3-Amino-1-hydroxypropan-1,1-diphosphonsäure, hergestellt nach DE 2,943,498, werden in 200 ml Wasser suspendiert und mit 20 ml n-Natronlauge bis pH⁹ versetzt. Dann gibt man unter Konstanthaltung des pH-Wertes 12,10 g (30 mMol) N³-(2,6-Dioxomorpholinoethyl)--N⁶-(ethoxycarbonylmethyl)-3,6-diazaoctandisäure zu und lässt über Nacht rühren. Dann bringt man die Lösung mit n-Natronlauge auf pH 12,5 und läßt 3 Stunden nachrühren. Nach Zusatz von Kationenaustauscher IR 120 bis pH 7 wird die Lösung filtriert und über Kieselgel chromatographiert (Laufmittel: Butanol/Ammoniak/Ethanol/Wasser = 5/2/1/1). Die vereinigten substanzhaltigen Eluate werden im Vakuum zur Trockne eingeengt. Man erhält 1,3 g eines weißen Pulvers vom Schmelzpunkt 145° C.

| Analyse (bezogen auf wasserfreie Substanz): | | | | |
|---|---|---|---|---|
| C 33,45 | H 5,28 | N 9,18 | P 10,15 | (berechnet) |
| C 33,56 | H 5,50 | N 9,30 | P 10,02 | (gefunden) |

### 5) Calcium-Trinatriumsalz der DTPA (CaNa₃DTPA).

196,6 g (0,5 Mol) DTPA werden mit 50 g (0,5 Mol) Calciumcarbonat in 800 ml Wasser bis zur Beendigung der Gasentwicklung zum Rückfluß erhitzt. Dann stellt man durch Zugabe von 750 ml einer 2 n-Natronlauge eine neutrale Salzlösung her (pH 7,1), die im Vakuum zur Trockne eingeengt wird. Nach Trocknen über Nacht im Vakuum erhält man 246,2 g des Komplexsalzes als Monohydrat mit einem bei 178-180° C -liegenden Schmelzpunkt.

| Analyse (bezogen auf wasserfreie Substanz): | | | | |
|---|---|---|---|---|
| C 35,45 | H 3,82 | N 8,86 | Ca 8,45 | (berechnet) |
| C 35,30 | H 3,96 | N 8,80 | Ca 8,39 | (gefunden) |

### Pharmakologische Untersuchungen

In pharmakologischen Prüfungen wurde festgestellt, daß ein zugesetzter Anteil an freien Komplexbildnern bzw. schwächerem Metallkomplex trotz absolut und relativ niedriger Dosierung die vollständige Ausscheidung des Schwermetallions ganz bedeutend fördert.

So zeigt Tabelle 1, daß durch Zusatz von nur 10 % Calciumtrinatrium DTPA zu dem Kontrastmittel der eine Woche nach i.v. Injektion von GdDTPA in den Körpern von Ratten verbliebene Gadoliniumanteil um>30 %, die Konzentration in den Knochen sogar um ca. 45 % vermindert wird.

**Tabelle 1**

| ¹⁵³Gd in der Ratte 7 Tage nach i.v.-Injektion als GdTPA/dimeglumin in einer Dosis von 0,1 mmol/kg, n = 3, Mittel ± Standardabweichung | | |
|---|---|---|
| | Formulierung A 0,1 mmol GdDTPA /kg KG | Formulierung B 0,1 mmol GdDTPA + 0,01 mmol CaNa₃DTPA/kg KG |
| Knochen (nmol/g) | 1,46 ± 0,21 | 0,79 ± 0,22 |
| Menge im Gesamttier (% der Dosis) | 0,99 ± 0,24 | 0,68 ± 0,09 |

Die Werte der Tabelle 2 zeigen, daß ein Zusatz von nur 2 Molprozent freies DTPA (Formulierung B) zu einem Kontrastmittel auf Basis von GdDTPA die Gadolinium-Konzentration in der Leber von Ratten gegenüber einer Kontrolle mit 0,08 Molprozent freier DTPA (Formulierung A) bis zum 28. Tag nach Injektion um mehr als 50 % vermindert.

**Tabelle 2**

| ¹⁵³Gd in der Leber der Ratte nach i.v. Injektion von GdDTPA/dimeglumin in einer Dosis von 0,5 mmol/kg; mmol/g Frischgewicht; n = 3; Mitte ± Standardabweichung | | | | | | | |
|---|---|---|---|---|---|---|---|
| | 2 h | 6 h | 1 d | 3 d | 7 d | 14 d | 28 d p.inj. |
| Formulierung A | | | | | | | |
| 0,5 mmol GdDTPA + 0,0004 mmol DTPA/kg | 20 ± 2 | 20 ± 3 | 10 ± 3 | 5,6 ±0,9 | 2,3 ±0,6 | 1,1 ±0,3 | 0,4 ±0,1 |

| Formulierung B | | | | | | | |
|---|---|---|---|---|---|---|---|
| 0,5 mmol GdDTPA + 0,01 mmol DTPA/kg | 22 ± 7 | 17 ± 1 | 10 ± 2 | 5,2 ±1,4 | 1,6 ±0,1 | 0,6 ±0,2 | 0,13 ±0,01 |

Dieser beobachtete Effekt des Zusatzes von Komplexbildner bzw. schwächerem Metallkomplex zu pharmazeutischen Präparaten auf der Basis von Metallkomplexen war in keinster Weise vorhersagbar; tatsächlich steht er sogar im Widerspruch zu entsprechenden in-vitro-Bestimmungen.

Eine Abschätzung der in-vivo-Situation wird durch die Tatsache praktisch unmöglich gemacht, daß alle Formen des Komplexbildners mit körpereigenen Ionen in Wechselwirkung treten können und sich andererseits für Schwermetalle zahlreiche, z.T. äußerst feste Bindestellen (Eiweiße; natürliche Komplexbildner; Anionen, die sehr wenig lösliche Salze bilden) anbieten. Zusätzlich mußte zur Lösung der Aufgabe, besser verträgliche diagnostische Mittel zur Verfügung zu stellen, ein Vorurteil der Fachwelt überwunden werden: Es wurde bisher bei der Herstellung von Kontrastmitteln auf der Basis von Metallkomplexen immer sorgfältig darauf geachtet, daß in der Lösung weder ein Überschuß an Metallionen noch ein Überschuß an freien Komplexbildnern bzw. schwächerem Komplex vorlag, da bekannt ist, daß sowohl die freien Komplexbildner wie auch die schwächeren Komplexe der Komplexbildner mit Metallionen wie z.B. Ca²⁺ schlechter verträglich sind als die stärkeren Komplexe mit den für die Diagnostik bzw. Therapie geeigneten Schwermetallionen.

Das Überraschende der vorliegenden Erfindung liegt daher auch in der außerordentlich starken Wirksamkeit des Zusatzes freier Komplexbildner oder schwächerer Komplexe zu dem für die Anwendung am Menschen vorgesehenen Metallkomplex im Hinblick auf die Stabilität der Bindung des Metalls und damit seiner Entgiftung und Ausscheidung. Dieser Vorteil ist angesichts der nur langsamen Ausscheidung von Schwermetallen und deren inherenter Toxizität von so großer Bedeutung, daß dafür unter Umständen sogar eine etwas verminderte akute Verträglichkeit eines Präparates in Kauf genommen werden kann.

Zur Erreichung des gewünschten Zweckes reichen häufig sehr geringe Konzentrationen und Dosierungen aus. Nach oben ist die Dosierung des Komplexbildners bzw. der schwächeren Komplexe durch deren akute Verträglichkeit begrenzt. Geeignet ist ein Bereich von 0,1 bis 10 Molprozent oder max. 50 mMol/l des eigentlichen diagnostisch wirksamen Komplexbildners.

Auf keinen Fall ist es notwendig, den Komplexbildner oder schwächeren Komplex so hoch dosiert den Präparaten zuzusetzen, daß es zu einer relevanten Einschränkung der Verträglichkeit des Fertigpräparates gegenüber dem ursprünglich ausgewählten, diagnostisch oder therapeutisch wirksamen Metallkomplex kommt. Außerdem ist keinesfalls eine isolierte oder gar noch wiederholte Verabreichung des Komplexbildners oder schwächeren Komplexes notwendig.

Die Herstellung der erfindungsgemäßen pharmazeutischen Mittel erfolgt in an sich bekannter Weise, indem man - gegebenenfalls unter Zugabe der in der Galenik üblichen Zusätze - die in wäßrigem Medium suspendierten oder gelösten Komplexverbindungen mit dem/den als Zusatz verwendeten komplexbildende(n) oder schwächerem/n Metall-Komplex(en) mischt und anschließend die Suspension oder Lösung gegebenenfalls sterilisiert. Geeignete Zusätze sind beispielsweise physiologisch unbedenkliche Puffer (wie z.B. Tromethamin), viskositätserhöhende Zusätze, solche, die die Osmolalität erhöhen oder, falls erforderlich, Elektrolyte wie z.B. Natriumchlorid oder, falls erforderlich, Antioxidantien wie z.B. Ascorbinsäure.

Sind für die enterale Verabreichung oder andere Zwecke Suspensionen oder Lösungen der erfindungsgemäßen Mittel in Wasser oder physiologischer Salzlösungen erwünscht, können sie mit einem oder mehreren in der Galenik üblichen Hilfsstoffen (z.B. Methylcellulose. Lactose, Mannit) und/oder Tensiden (z.B. Lecithine, Tweens^{(R)}, Myrj^{(R)} und/oder Aromastoffen zur Geschmackskorrektur (z.B. ätherischen Ölen) gemischt werden.

Sind für die orale Verabreichung oder andere Zwecke Suspensionen der Komplexverbindungen in Wasser oder physiologische Salzlösung erwünscht, wird eine wenig lösliche Komplexverbindung mit einem oder mehreren in der Galenik üblichen Hilfsstoffen und/oder Tensiden und/oder Aromastoffen zur Geschmackskorrektur gemischt.

Die erfindungsgemäßen pharmazeutischen Mittel enthalten vorzugsweise 1 µMol - 1 Mol/l des Komplexsalzes und werden in der Regel in Mengen von 0.001 - 5 mMol/kg dosiert. Sie sind zur enteralen und parenteralen Applikation bestimmt. Ihre Herstellung wird in den nachfolgenden Beispielen näher beschrieben.

### Beispiel 1

Herstellung einer Lösung des Di-N-methylglucaminsalzes des Gadolinium-(III)-komplexes der Diethylentriaminpentaessigsäure (DTPA) mit dem Calcium-Trinatriumsalz der DTPA als Zusatz.

97,6 g (0,5 Mol) N-Methylglucamin werden in 50 ml Wasser pro injectione (p.i.) gelöst. Nach Zugabe von 196,6 g (0,5 Mol) DTPA und 90,6 g (0,25 Mol) Gadoliniumoxid, Gd₂O₃, erhitzt man zwei Stunden zum Rückfluß und bringt die klare Lösung durch Zugabe von nochmals 97,6 kg (0,5 Mol) N-Methylglucamin auf pH 7,2. Nun setzt man 24,62 g (50 mMol) Monohydrat des Calcium-Trinatriumsalzes der DTPA, CaNa₃ DTPA, zu und füllt mit Wasser p.i. auf 1000 ml auf. Die Lösung wird ultrafiltriert, ampulliert und hitzesterilisiert und ist für die parenterale Anwendung gebrauchsfertig.

### Beispiel 2

Herstellung einer Lösung des Di-N-methylglucaminsalzes des Gadolinium-(III)-Komplexes der Diethylentriaminpentaessigsäure (DTPA) mit dem Penta-N-methylglucaminsalz der DTPA als Zusatz.

97,6 g (0,5 Mol) N-Methylglucamin werden in 500 ml Wasser pro injectione (p.i.) gelöst. Nach Zugabe von 196,6 g (0,5 Mol) DTPA und 90,6 g (0,25 Mol) Gadoliniumoxid. Gd₂O₃, erhitzt man den Ansatz zwei Stunden zum Rückfluß und bringt die klare Lösung durch Zugabe von nochmals 97,6 g (0,5 Mol) N-Methylglucamin auf pH 7,2. Nun fügt man noch eine Lösung von 197 mg (0,5 mMol) DTPA und 488 mg (2,5 mMol) N-Methylglucamin in 100 ml Wasser p.i. zu und füllt mit Wasser p.i. auf 1000 ml auf. Die Lösung wird abschließend ultrafiltriert, ampulliert und hitzesterilisiert und ist für die parenterale Anwendung gebrauchsfertig.

### Beispiel 3

Herstellung einer Lösung des Natriumsalzes des Gadolinium-(III)-Komplexes der 1,4,7,10 - Tetraazacyclododecan-N,N',N'',N'''-tetraessigsäure (DOTA) mit dem Calcium-Dinatriumsalz der DOTA als Zusatz.

290,3 g (0,5 Mol) des im Beispiel 11 der DE 3401052 beschriebenen Komplexsalzes werden in 700 ml Wasser p.i. gelöst. Nach Zugabe von 7,44g (30 mMol) Calcium-Dinatriumsalz der DOTA wird die neutrale Lösung mit Wasser p.i. auf 1000 ml aufgefüllt, ultrafiltriert, ampulliert und hitzesterilisiert.

### Beispiel 4

Herstellung einer Lösung des Lysinsalzes des Gadolinium-(III)-Komplexes der DOTA mit dem Zink-Dinatriumsalz der DOTA als Zusatz.

80,80 g (0,2 Mol) DOTA (Parish Chemical Comp.) werden in eine Suspension von 36,26 g (0,1 Mol) Gadoliniumoxid, Gd₂O₃, in 700 ml Wasser p.i. eingetragen. Man erwärmt unter Rühren 20 Stunden auf 70° C und neutralisiert die Lösung durch Zugabe einer wässrigen 20 gew.-%igen Lösung von Lysin. Dann setzt man 10,24 g (20 mMol) Zink-Dinatrium-Salz der DOTA zu und füllt die Lösung mit Wasser p.i. auf 1000 ml auf. Die Lösung wird ultrafiltriert, ampulliert und hitzesterilisiert.

### Beispiel 5

Herstellung einer Lösung des Di-N-methylglucaminsalzes des Gadolinium-(III)-Komplexes der DTPA mit dem Calcium-Trinatriumsalz der DTPA als Zusatz.

97,6 g (0,5 Mol) N-Methylglucamin werden in 20 l Wasser gelöst. Nach Zugabe von 196.6 g (0,5 Mol) DTPA und 90,6 g (0,25 Mol) Gadoliniumoxid Gd₂O₃, erhitzt man 5 Stunden zum Rückfluß. Man setzt 475,4 g (1 Mol) Calcium-Trinatrium-DTPA, 750 g Mannit und 100 g Trinatriumcitrat zu, neutralisiert die Lösung mit N-Methylglucamin und füllt mit Wasser auf 50 l auf. Die Lösung wird für die enterale Anwendung in Flaschen abgefüllt.

### Beispiel 6

Herstellung einer Lösung des Gadolinium(III)-Komplexes der N⁶-Carboxymethyl-N³,N⁹-bis-(methylcarbamoylmethyl)-3,6,9--triazaundecandisäure mit N⁶-Carboxymethyl-N³,N⁹-bis-(methylcarbamoylmethyl)-3,6,9-triazaundecandisäure als Zusatz.

12,58 g (30 mMol) N⁶-Carboxymethyl-N³,N⁹-bis(methylcarbamoylmethyl)-3,6,9-triazaundecandisäure werden mit 5,44 g (15 mMol) Gadoliniumoxid. Gd₂O₃, in 500 ml Wasser 6 Stunden bei 90° C zur Reäktion gebracht. Man setzt 12.58 g (30 mMol) N⁶-Carboxymethyl-N³,N⁹-bis (methylcarbamoylmethyl) -3,6,9-triazaundecandisäure, 50 g Mannit und 8 g Trinatriumcitrat zu; neutralisiert die Lösung mit N-Methylglucamin und füllt mit Wasser auf 1000 ml auf. Die Lösung wird für die enterale Anwendung in Flaschen abgefüllt.

### Beispiel 7

Herstellung einer Lösung des Gadolinium-(III)-Komplexes der N⁶-Carboxymethyl-N³,N⁹-bis (3-oxapentamethylencarbmoylmethyl)-3,6,9-triazaundecandisäure mit dem Tetranatriumsalz der N³, N⁶-Bis-(carboxymethyI)-N⁹-3-oxapentamethylen-carbamoylmethyl-3,6,9-triazaundecandisäure als Zusatz.

17,90 g (50 mMol) 1,5-Bis-(2,6-dioxomorpholino)-3-azapentan-9-essigsäure werden in 150 ml Wasser suspendiert und durch Zugabe von 13,08 ml (150 mMol) Morpholin gelöst. Man läßt 16 Stunden bei Raumtemperatur rühren und versetzt mit 16,57 (50 mMol) Gadolinium-(III)-acetat, gelöst in 150 ml Wasser. Man läßt 2 Stunden nachrühren und behandelt die Lösung nacheinander mit Anionenautauscher IRA 410 und Kationenaustauscher IRC 50. Die neutrale Lösung wird anschließend im Vakuum zur Trockne eingeengt. Man erhält 22,25 g (32,4 mMol) der gewünschten Komplexverbindung, die in einer Lösung von 12,65 g (64,8 mMol)N-Methylglucamin in 60 ml Wasser p.i. aufgelöst werden. Man setzt 1,66 g (3 mMol) Tetranatriumsalz der N³,N⁶-Bis-(carboxymethyl)-N⁹-3-oxapentamethylen-carbamoylmethyl-3,6,9-triazaundecandisäure zu und füllt auf 100 ml mit Wasser p.i. auf. Die neutrale Lösung wird ultrafiltriert, in Ampullen abgefüllt und hitzesterilisiert; sie ist für die parenterale Anwendung gebrauchsfertig.

### Beispiel 8

Herstellung einer Lösung des Dinatriumsalzes des Mangan(II)-Komplexes der trans-1,2-Cyclohexylendiamintetraessigsäure mit trans-1,2-Cyclohexylendiamintetraessigsäure als Zusatz.

395,9 g (500 mMol) des im Beispiel 9 der DE 3401052 beschriebenen Salzes werden in 500 ml Wasser p.i. unter Stickstoffbegasung suspendiert. Man versetzt mit 1,73 g Ascorbinsäure und 17,3 g (50 mMol) trans-1,2-Cyclohexylendiamintetraessigsäure, dann neutralisiert man durch Zugabe von n-Natronlauge und füllt mit Wasser p.i. auf 1000 ml auf. Die Lösung wird unter Stickstoff sterilfiltriert und in Ampullen abgefüllt.

### Beispiel 9

Herstellung einer Lösung des N-Methylglucaminsalzes des Eisen(III)-komplexes der Ethylendiamin-N,N'-bis (2-hydroxyphenylessigsäure) (EHPG) mit EHPG als Zusatz.

36,04 g (0,1 Mol) EHPG werden in 500 ml Wasser p.i. mit 15,97 g (0,1 Mol) Eisen(III)-oxid, Fe₂O₃, solange zum Rückfluß erhitzt, bis Lösung eingetreten ist, Man setzt 3,604 g (0,01 Mol) EHPG zu und bringt durch Zugabe von N-Methylglucamin auf pH 7,5. Man füllt mit Wasser p.i. auf 1000 ml auf und ultrafiltriert die Lösung, die anschließend in Ampullen abgefüllt und hitzesterilisiert wird.

### Beispiel 10

Herstellung einer Injektionslösung des Natriumsalzes des Gadolinium-(III)-komplexes der N³,N⁶-bis-(carboxymethyl)-N⁹[3,3-bis(dihydroxyphosphoryl)-3-hydroxy-propylcarbamoylmethyl]-3,6,9-triazaundecandisäure mit N³N⁶-Bis(carboxmethyl)-N⁹ [3,3-bis(dihydroxyphosphoryl)-3-hydroxypropylcarbamoylmethyl] -3,6,9-triazaundecandisäure als Zusatz.

305,2g (0,5 Mol)N³, N⁶-bis (carboxymethyl)-N⁹- [3,3-bis(dihydroxyphosphoryl)3-hydroxypropylcarbamoylmethyl]-3,6,9-triazaundecandisäure werden in 8,5 l Wasser p.i. unter Zusatz von 123,6 g (0,25 Mol) Gadoliniumcarbonat, Gd₂(Co₃)₃ bei 50° C gelöst. Dann setzt man nochmals 30,52 g (0,05 Mol) des Komplexbildners zu und bringt unter Stickstoffbegasung durch Zutropfen von 5 n-Natronlauge auf pH 7,2, die Lösung wird mit Wasser p.i. auf 10 l aufgefüllt, ultrafiltriert, in Ampullen angefüllt und hitzesterilisiert.

### Beispiel 11

Herstellung einer Lösung des Gadolinium(III)-Komplexes vom Konjugat der DTPA mit monoklonalem Antikörper mit dem Calcium-Trinatriumsalz der DTPA als Zusatz.

Zu 20 µl einer Lösung von 0,3 mg des monoklonalen Antikörpers 12 H 12 in 0,05 molarem Natriumbicarbonatpuffer werden 1 mg N³-(2,6-Dioxomorpholinoethyl)-N⁶-(ethoxycarbonylmethyl)-3,6-diazaoctandisäure zugegeben. Nach Rührer über Nacht versetzt man mit 2 mg Gadoliniumchlorid, GdCl₃, und dialysiert die Lösung gegen einen 0,3 molaren Natriumphosphatpuffer. Dann setzt man 1 mg Calcium-Trinatriumsalz der DTPA zu. Die sterilfiltrierte Lösung wird in Multivials abgefüllt und lyophilisiert.

### Beispiel 12

Herstellung einer Lösung des Gadolinium-(III)-Komplexes des Dextran-DTPA-Konjugats mit dem Calcium-Dinatriumsalz der EDTA als Zusatz.

Eine Lösung von 6,5 g Dextran 10000 wird mit n-Natronlauge auf pH 11 gebracht. Dann setzt man 2,12 g Bromcyan zu und läßt 30 Minuten rühren, wobei der pH durch weitere Laugenzugabe konstant gehalten wird. Nach Zugabe von 20 g 1-(4-Aminobenzyl)DTPA, hergestellt nach Can.Pat. 1178951, läßt man die Lösung bei pH 8.5 über Nachtnachrühren. Dann wird eine Lösung von 8 g Gadoliniumchlorid, GdCl₃, in 30 ml Wasser zugetropft, die Lösung über Aktivkohle geklärt und dialysiert. Das Dialysat wird mit 3 g Calcium-Dinatriumsalz der Ethylendiamintetraessigsäure (EDTA) versetzt, sterilfiltriert, in Multivials abgefüllt und lyophilisiert. Das Lyophilisat enthält ca. 10 % komplexgebundenes Gadolinium.

## Patentansprüche

1. Verwendung eines oder mehrerer Komplexbildner und/oder eines oder mehrerer schwächeren Metall-Komplexe oder Gemische von ihnen in einer Menge von 0,1-10 Mol % oder max. 50mMol/l als Additiv bei der Herstellung von pharmazeutischen Mitteln für die NMR-Diagnostik enthaltend mindestens eine für die NMR-Diagnostik geeignete, ein paramagnetisches Metall, ausgenommen Chrom, enthaltende Komplexverbindung zur Verbesserung von deren Verträglichkeit.

2. Verwendung gemäß Anspruch 1, dadurch gekennzeichnet, daß der/die schwächere(n) Metall-Komplex(e) (ein) Metallion(en) aus der Reihe der Elemente Calcium, Magnesium, Zink und Eisen enthält/enthalten.

3. Verwendung des Calcium-Trinatriumsalzes der DTPA gemäß Anspruch 1 in Mitteln enthaltend das Di-N-methylglucaminsalz des Gadolinium(III)-Komplexes der DTPA.

4. Verwendung des Penta-N-methylglucaminsalzes der DTPA gemäß Anspruch 1 in Mitteln enthaltend das Di-N-methylglucaminsalz des Gadolinium(III)-Komplexes des DTPA.

5. Verwendung des Calcium-Dinatriumsalzes der DOTA gemäß Anspruch 1 in Mitteln enthaltend das Natriumsalz des Gadolinium(III)-Komplexes der 1,4,7,10-Tetraazacyclododecan-N,N'-N'',N'''-tetraessigsäure (DOTA).

6. Verwendung des Zink-Dinatriumsalzes der DOTA gemäß Anspruch 1 in Mitteln enthaltend das Lysinsalz des Gadolinium(III)-Komplexes der DOTA.

7. Verwendung der N⁶-Carboxymethyl-N³,N⁹-bis-(methylcarbamoylmethyl)-3,6,9-triazaundecandisäure gemäß Anspruch 1 in Mitteln enthaltend den Gadolinium(III)-Komplex der N⁶-Carboxymethyl-N³,N⁹-bis-(methylcarbamoylmethyl)-3,6,9-triazaundecandisäure.

8. Verwendung des Tetranatriumsalzes der N³,N⁶-bis-(carboxymethyl)-N⁹-3-oxapentamethylen-carbamoylmethyl-3,6,9-triazaundecandisäure gemäß Anspruch 1 in Mitteln enthaltend den Gadolinium(III)-Komplex der N⁶-carboxymethyl-N³,N⁹-bis-(3-oxapentamethylen-carbamoylmethyl)-3,6,9-triazaundecandisäure.

9. Verwendung der trans-1,2-Cyclohexylendiamintetraessigsäure gemäß Anspruch 1 in Mitteln enthaltend das Di-Natriumsalz des Mangan(II)-Komplexes der trans-1,2-Cyclohexylendiamintetraessigsäure.

10. Verwendung der Ethylendiamin-N,N'-bis-(2-hydroxyphenylessig-säure) (EHPG) gemäß Anspruch 1 in Mitteln enthaltend das N-Methylglucaminsalz des Eisen(III)-Komplexes von EHPG.

11. Verwendung der N³,N⁶-Bis(carboxymethyl)-N⁹-[3,3-bis-(dihydroxyphosphoryl)-3-hydroxypropylcarbamoylmethyl]-3,6,9-triazaundecandisäure gemäß Anspruch 1 in Mitteln enthaltend das Natriumsalz des Gadolinium(III)-Komplexes der N³,N⁶-Bis(carboxymethyl)-N⁹-[3,3-bis-(dihydroxyphosphoryl)-3-hydroxypropylcarbamoylmethyl]-3,6,9-triazaundecandisäure.

12. Verwendung des Calcium-Dinatriumsalzes der DTPA gemäß Anspruch 1 in Mitteln enthaltend den Gadolinium(III)-Komplex vom Konjugat der DTPA mit monoklonalem Antikörper.

13. Verwendung des Calcium-Dinatriumsalzes der EDTA gemäß Anspruch 1 in Mitteln enthaltend den Gadolinium(III)-Komplex des Dextran-DTPA-Konjugats.

## Claims

1. Use of one or more complexing agent(s) and/or of one or more weaker metal complex(es), or mixtures thereof, in an amount of from 0.1 to 10 mol % or a maximum of 50 mmol/l as additive in the manufacture of pharmaceutical agents for NMR diagnostics containing at least one complex compound suitable for NMR diagnostics that contains a paramagnetic metal, with the exception of chromium, for improving the tolerability thereof.

2. Use according to claim 1, characterised in that the weaker metal complex(es) contain(s) (a) metal ion(s) from the group of elements calcium, magnesium, zinc and iron.

3. Use of the calcium trisodium salt of DTPA according to claim 1 in agents containing the di-N-methylglucamine salt of the gadolinium(III) complex of DTPA.

4. Use of the penta-N-methylglucamine salt of DTPA according to claim 1 in agents containing the di-N-methylglucamine salt of the gadolinium(III) complex of DTPA.

5. Use of the calcium disodium salt of DOTA according to claim 1 in agents containing the sodium salt of the gadolinium(III) complex of 1,4,7,10-tetraazacyclododecane-N,N'-N'',N'''-tetraacetic acid (DOTA).

6. Use of the zinc disodium salt of DOTA according to claim 1 in agents containing the lysine salt of the gadolinium(III) complex of DOTA.

7. Use of N⁶-carboxymethyl-N³,N⁹-bis-(methylcarbamoylmethyl)-3,6,9-triazaundecanedioic acid according to claim 1 in agents containing the gadolinium(III) complex of N⁶-carboxymethyl-N³,N⁹-bis-(methylcarbamoylmethyl)-3,6,9-triazaundecanedioic acid.

8. Use of the tetrasodium salt of N³,N⁶-bis-(carboxymethyl)-N⁹-3-oxapentamethylenecarbamoylmethyl-3,6,9-triazaundecanedioic acid according to claim 1 in agents containing the gadolinium(III) complex of N⁶-carboxymethyl-N³,N⁹-bis-(3-oxapentamethylenecarbamoylmethyl)-3,6,9-triazaundecanedioic acid.

9. Use of trans-1,2-cyclohexylenediaminetetraacetic acid according to claim 1 in agents containing the disodium salt of the manganese(II) complex of trans-1,2-cyclohexylenediaminetetraacetic acid.

10. Use of the ethylenediamine-N,N'-bis-(2-hydroxyphenylacetic acid) (EHPG) according to claim 1 in agents containing the N-methylglucamine salt of the iron(III) complex of EHPG.

11. Use of N³,N⁶-bis-(carboxymethyl)-N⁹-[3,3-bis-(dihydroxyphosphoryl)-3-hydroxypropylcarbamoylmethyl]-3,6,9-triazaundecanedioic acid according to claim 1 in agents containing the sodium salt of the gadolinium(III) complex of N³,N⁶-bis-(carboxymethyl)-N⁹-[3,3-bis-(dihydroxyphosphoryl)-3-hydroxypropylcarbamoylmethyl]-3,6,9-triazaundecanedioic acid.

12. Use of the calcium disodium salt of DTPA according to claim 1 in agents containing the gadolinium(III) complex of the conjugate of DTPA with monoclonal antibody.

13. Use of the calcium disodium salt of EDTA according to claim 1 in agents containing the gadolinium(III) complex of the dextran-DTPA conjugate.

## Revendications

1. Utilisation d'un ou de plusieurs agents complexants et/ou d'un ou de plusieurs complexes à métaux plus faibles ou de leurs mélanges dans une quantité de 0,1 à 10 % en moles ou de 50 mmoles/l au maximum comme adjuvant dans la préparation d'agents pharmaceutiques pour le diagnostic par la RMN, contenant au moins un composé complexe, approprié au diagnostic par la RMN, contenant un métal paramagnétique, à l'exception du chrome, pour améliorer leur compatibilité.

2. Utilisation selon la revendication 1, caractérisée en ce que le/les complexe(s) à métaux plus faible(s) contient/contiennent un (des) ion(s) métallique(s) de la série des éléments calcium, magnésium, zinc et fer.

3. Utilisation du sel trisodique et calcique du DTPA selon la revendication 1 dans des agents contenant le sel de la di-N-méthylglucamine du complexe de gadolinium(III) du DTPA.

4. Utilisation du sel de la penta-N-méthylglucamine du DTPA selon la revendication 1 dans des agents contenant le sel de la di-N-méthylglucamine du complexe de gadolinium(III) du DTPA.

5. Utilisation du sel calcique et disodique du DOTA selon la revendication 1 dans des agents contenant le sel sodique du complexe de gadolinium(III) de l'acide 1,4,7,10-tétraazacyclododécane-N,N',N'',N'''-tétraacétique (DOTA).

6. Utilisation du sel zincique et disodique du DOTA selon la revendication 1 dans des agents contenant le sel de la lysine du complexe de gadolinium(III) du DOTA.

7. Utilisation de l'acide N⁶-carboxyméthyl-N³,N⁹-bis-(méthylcarbamoylméthyl)-3,6,9-triazaundécane-dioïque selon la revendication 1 dans des agents contenant le complexe de gadolinium(III) de l'acide N⁶-carboxyméthyl-N³,N⁹-bis-(méthylcarbamoylméthyl)-3,6,9-triazaundécanedioïque.

8. Utilisation du sel tétrasodique de l'acide N³,N⁹-bis-(carboxyméthyl)-N⁹-3-oxapentaméthylène-carbamoylméthyl-3,6,9-triazaundécane-dioïque selon la revendication 1 dans des agents contenant le complexe de gadolinium(III) de l'acide N⁶-carboxyméthyl-N³,N⁹-bis-(3-oxapentaméthylèneméthyl-carbamoyl)-3,6,9-triaza-undécane-dioïque.

9. Utilisation de l'acide trans-1,2-cyclohexylènediamine-tétraacétique selon la revendication 1 dans des agents contenant le sel disodique du complexe du manganèse (II) de l'acide trans-1,2-cyclohexylène-diamine-tétraacétique.

10. Utilisation de l'acide éthylène-diamine-N,N'-bis-(2-hydroxyphénylacétique) (EHPG) selon la revendication 1 dans des agents contenant le sel de la N-méthylglucamine du complexe de fer(III) de l'EHPG.

11. Utilisation de l' acide N³,N⁹-bis-(carboxyméthyl)-N⁹-[3,3-bis-(dihydroxyphosphoryl)-3-hydroxypropyl-carbamoyl-méthyl]-3,6,9-triazaundécane-dioïque selon la revendication 1 dans des agents contenant le sel sodique du complexe de gadolinium(III) de l'acide N³,N⁹-bis-(carboxyméthyl)-N⁹-[3,3-bis-(dihydroxyphosphoryl)-3-hydroxypropyl-carbamoyl-méthyl]-3,6,9-triazaundécanedioïque.

12. Utilisation du sel calcique et disodique du DTPA selon la revendication 1, dans des agents contenant le complexe de gadolinium(III) du conjugué du DTPA avec des anticorps monoclonaux.

13. Utilisation du sel calcique et disodique de l'EDTA selon la revendication 1, dans des agents contenant le complexe de gadolinium du conjugué dextranne-DTPA.
